(19) Europäisches Patentamt
European Patent Office
Office européen des brevets

(11) Veröffentlichungsnummer : **0 402 806 B1**

(12) **EUROPÄISCHE PATENTSCHRIFT**

(45) Veröffentlichungstag der Patentschrift : **29.09.93 Patentblatt 93/39**

(51) Int. Cl.$^5$ : **C07D 501/34,** A61K 31/545

(21) Anmeldenummer : **90110945.4**

(22) Anmeldetag : **09.06.90**

(54) **Kristalline Cephem Säureadditionssalze und Verfahren zu ihrer Herstellung.**

(30) Priorität : **13.06.89 DE 3919259**

(43) Veröffentlichungstag der Anmeldung : **19.12.90 Patentblatt 90/51**

(45) Bekanntmachung des Hinweises auf die Patenterteilung : **29.09.93 Patentblatt 93/39**

(84) Benannte Vertragsstaaten : **AT BE CH DE DK ES FR GB GR IT LI LU NL SE**

(56) Entgegenhaltungen :
**EP-A- 0 049 119**
**EP-A- 0 134 420**

(73) Patentinhaber : **HOECHST AKTIENGESELLSCHAFT**
**D-65926 Frankfurt (DE)**

(72) Erfinder : **Adam, Friedhelm, Dr.**
**Rheingaustrasse 46**
**D-6238 Hofheim am Taunus (DE)**
Erfinder : **Dürckheimer, Walter, Dr.**
**Im Lerchenfeld 45**
**D-6234 Hattersheim am Main (DE)**
Erfinder : **Mencke, Burkhard, Dr.**
**Haupstrasse 2**
**D-5409 Holzappel (DE)**
Erfinder : **Isert, Dieter, Dr.**
**Hamburger Strasse 1**
**D-6236 Eschborn (DE)**

EP 0 402 806 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind kristalline, enteral resorbierbare Salze des 7-[2-(2-Aminothiazol-4-yl)-2-(Z) hydroxyimino-acetamido]-3-methoxymethyl-3-cephem-4- carbonsäure-α-(2,2-dimethyl-propanoyloxy)-ethylesters der Formel I

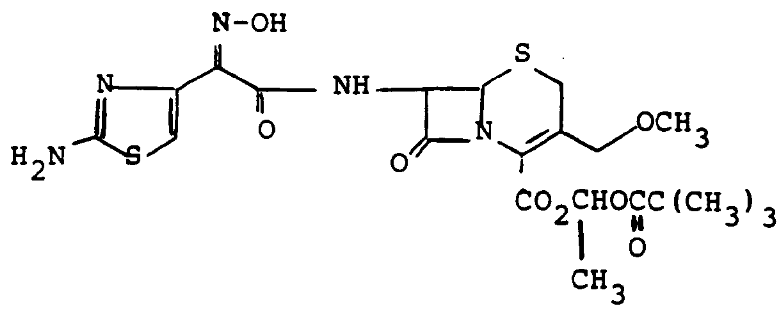

(I)

sowie ein Verfahren zu ihrer Herstellung.

Viele klinisch relevante Cephalosporine mit breitem antimikrobiellem Spektrum sind bereits entwickelt worden. Die meisten eignen sich jedoch nur für eine parenterale Anwendung, da sie nach oraler Gabe, wenn überhaupt, nur sehr unzureichend resorbiert werden. In vielen Fällen ist es jedoch wünschenswert, dem Patienten hochwirksame Antibiotika in oraler Form zu geben. Diese Form der Therapie ist einfacher und senkt erheblich die Kosten der Behandlung.

In einigen Fällen ist es gelungen, die Resorption eines Cephalosporins im Gastrointenstinaltrakt durch Veresterung der 4-Carboxygruppe zu erhöhen. Da die Cephalosporinester in der Regel per se keine antibiotische Wirksamkeit aufweisen, muß die Esterkomponente so gewählt werden, daß der Ester nach der Resorption durch körpereigene Enzyme, wie Esterasen, wieder rasch und vollständig zum Cephalosporin mit freier Carboxylgruppe zurückgespalten wird.

In den deutschen Patentanmeldungen P 38 04 841 und P 38 09 561 werden eine Anzahl von Estern der 7-[2-(2-Aminothiazol-4-yl)-2-(Z)-hydroxyimino-acetamido]-3-methoxymethyl-3-cephem-4-carbonsäure beschrieben, die in verschiedenen Tierspezies sehr gut enteral resorbiert werden. Von den in der deutschen Patentanmeldung P 38 04 841 beschriebenen Estern wurde gefunden, daß der Ester der Formel I von besonderem Interesse ist.

Dieser Ester besitzt ein asymmetrisches Kohlenstoffatom in 1-Stellung der 1-Pivaloylgruppe und kann deshalb in Form von R- und S-Isomeren oder Mischungen davon existieren. Die Verfahren zur Herstellung des Esters der Formel I, die in der deutschen Patentanmeldung P 38 04 841 beschrieben sind, erzeugen das Material in amorpher Form. Die beiden Diastereomeren besitzen die gleiche Resorption.

Versuche zur Kristallisation von I aus den üblichen organischen Lösungsmitteln führten zu Verlusten. Außerdem wird das Verhältnis der beiden Diastereomeren, die verschiedene Löslichkeiten haben, beim Kristallisationsschritt verschoben.

Die Herstellung eines Salzes von I gelingt aufgrund der schwachen Basizität der Aminogruppe nur, wenn man den Ester der Formel I mit starken Säuren umsetzt. Die in der Patent- und sonstiger Literatur beschriebenen Verfahren zur Herstellung kristalliner Salze der verschiedensten Cephalosporinester führten bei ihrer Anwendung auf die obige Verbindung nicht zum gewünschten Ergebnis. So führt beispielsweise die Herstellung eines Hydrochlorids, Sulfats oder Phosphats nur zu einem amorphen Produkt.

Es war daher überraschend, daß erfindungsgemäß kristalline Produkte erhalten wurden.

Die Herstellung eines kristallinen Esters oder eines kristallinen Salzes, das beide Isomeren in einem annähernden 1:1-Verhältnis enthält, ist aber sehr erstrebenswert. Eine solche Operation führt zu einer Verbesserung der Reinheit, verbesserter Stabilität der labilen β-Lactam-Ester. Die Reinheit läßt sich anhand physikochemischer Parameter bestimmen, wie z.B. hoher Schmelzpunkt, Löslichkeit, Stabilität, Δ3/Δ2-Isomerisierung der Doppelbindung. Auch ist es auf diesem Weg möglich, in den Fällen, in denen Lösungsmittel oder andere bei der Salzherstellung verwendete Stoffe in das Kristallgitter eingelagert oder absorbiert werden, Produkte definierter oder reproduzierbarer Zusammensetzung zu erhalten.

Gegenstand der Erfindung sind kristalline, stöchiometrische und enteral resorbierbare Salze der allgemeinen Formel II

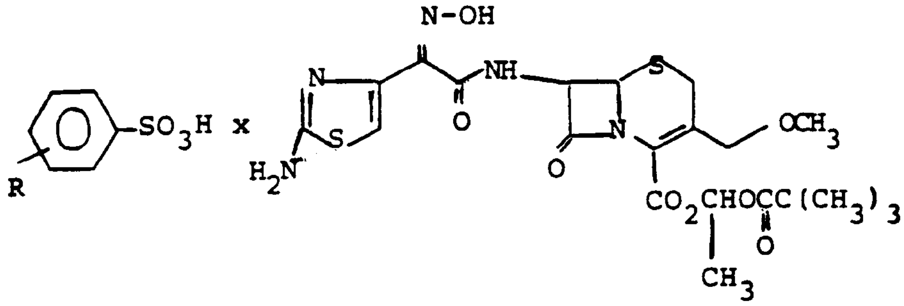

(II)

in der R für Wasserstoff oder $C_1$-$C_4$-Alkyl und die Gruppe =N-OH in syn-Position steht.

Besitzt R die Bedeutung von $C_1$-$C_4$-Alkyl, so kann es stehen z.B. für Methyl, Ethyl, Propyl, Isopropyl, Butyl, Isobutyl oder tert.-Butyl. Bevorzugt sind für R die Bedeutungen von Wasserstoff, Methyl oder Ethyl, insbesondere von Methyl oder Ethyl. R kann ortho-, meta- oder paraständig, bevorzugt aber paraständig sein. Als bevorzugte Säureadditionssalze kommen in Betracht das Benzolsulfonat, Toluolsulfonat oder p-Ethylbenzosulfonat.

Gegenstand der Erfindung ist weiterhin ein Verfahren zur Herstellung von Verbindungen der allgemeinen Formel II, das dadurch gekennzeichnet ist, daß man amorphes Material der Formel I mit Säuren der allgemeinen Formel III,

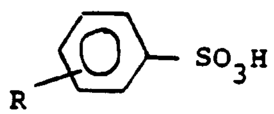

(III)

in der R die obige Bedeutung hat, in einem organischen Lösungsmittel umsetzt und diese Lösung - falls erforderlich - mit einem anderen organischen Lösungsmittel oder mit Wasser versetzt, um die Kristallisation einzuleiten und/oder zu vervollständigen.

Die Säurekomponente kann in geringem Überschuß, bevorzugt 1 - 1.5 Äquivalente bezogen auf den Ester der Formel I zur Anwendung kommen.

Die Wahl des Lösungsmittels erweist sich als bedeutend. Um ein annäherndes 1:1-Verhältnis der beiden Diastereomeren zu erhalten, muß ein Lösungsmittelsystem gewählt werden, das eine praktisch quantitative Gewinnung der Salze gewährleistet.

Für die Umsetzung kommen mit Wasser nicht mischbare organische Lösungsmittel wie z.B. Essigester, Essigsäure-n-butylester oder Essigsäure-tert.-butylester in Betracht, vorzugsweise jedoch solche, die - im Hinblick auf eine später eventuell erforderliche Wasserzugabe - mit Wasser mischbar sind.

Als organische, mit Wasser mischbare Lösungsmittel kommen z.B. in Betracht $C_1$-$C_4$-Alkohole, wie z.B. Methanol, Ethanol, Isopropanol, Propanol, Butanol, 2-Butanol, Isobutanol, tert.-Butanol, Aceton, Tetrahydrofuran oder Gemische derselben. Besonders bevorzugt sind Ethanol, Propanol und Aceton. Zur Vervollständigung der Kristallisation kann auch ein mit Wasser nicht mischbares Lösungsmittel, wie beispielsweise n-Hexan, Toluol, Diethylether oder Diisopropylether zu der Suspension der Kristalle in einem Gemisch aus Wasser und dem mit Wasser mischbaren Lösungsmittel gegeben werden. Eine solche Lösungsmittelzugabe ist natürlich auch möglich, wenn die Umsetzung in einem mit Wasser nicht mischbaren organischen Lösungsmittel durchgeführt wurde.

Bei der erfindungsgemäß bevorzugten Wasserzugabe beträgt die Menge des insgesamt zugefügten Wassers beispielsweise bis zum etwa 20-fachen des Volumens der organischen Lösung, sie kann aber auch noch wesentlich höher liegen.

Die Vereinigung der organischen Lösung mit dem Wasser oder mit einem weiteren organischen Lösungsmittel erfolgt langsam, z.B. tropfenweise, in dem Maße, daß eine gute Kristallinität des Produktes erreicht wird.

Die Kristallisation erfolgt vorzugsweise bei Raumtemperatur. Aber auch bei Temperaturen von beispielsweise 0 bis 40°C erhält man gute Ergebnisse. Eine Nachrührzeit von bis zu etwa 3 Stunden oder mehr vervollständigt die Kristallisation.

Die so erhaltenen kristallinen Salze der Formel II werden nach üblichen Laborverfahren, wie z.B. Filtration, abgetrennt und unter schwachem Vakuum von anhaftendem Lösungsmittel befreit.

Setzt man die durch Filtration gewonnenen Kristalle einem Hochvakuum (<1 Torr) aus, wird sowohl organisches Lösungsmittel als auch Wasser, besonders in Gegenwart eines Trocknungsmittels, wie z.B. konzentrierter Schwefelsäure, Phosphorsäureanhydrid, oder auch Ätzkali oder Ätznatron, sowie Silicagel entfernt.

Mit den kristallinen Salzen der Formel II durchgeführte Stabilitätsuntersuchungen zeigen gegenüber der Base I eine wesentliche Verbesserung der Stabilität.

Versuche an verschiedenen Tierspezies haben gezeigt, daß die Salze ebenfalls ausgezeichnet enteral resorbiert werden.

Die erfindungsgemäßen Verbindungen der allgemeinen Formel II werden oral verabreicht in Form von üblichen pharmazeutischen Zubereitungen, wie z.B. Kapseln, Tabletten, Pulvern, Sirupen oder Suspensionen. Die Dosis hängt ab vom Alter, den Symptomen und dem Körpergewicht des Patienten sowie von der Dauer der Behandlung. Sie liegt jedoch in der Regel zwischen etwa 0,2 g und etwa 5 g täglich, vorzugsweise zwischen etwa 0,5 g und etwa 3 g täglich. Die Verbindungen werden vorzugsweise in aufgeteilten Dosen verabreicht, beispielsweise 2 bis 4-mal täglich, wobei die Einzeldosis beispielsweise zwischen 50 und 500 mg Wirkstoff enthalten kann.

Die oralen Zubereitungen können die üblichen Trägerstoffe und/oder Verdünnungsmittel enthalten. So kommen beispielsweise für Kapseln oder Tabletten in Betracht Bindemittel, wie z.B. Gelatine, Sorbitol, Polyvinylpyrrolidon oder Carboxymethylcellulose, Verdünnungsmittel, wie z.B. Lactose, Zucker, Stärke, Calciumphosphate oder Polyethylenglykol, Gleitstoffe, wie z.B. Talkum oder Magnesiumstearat, für flüssige Zubereitungen, z.B. wäßrige oder ölige Suspensionen, Sirupe oder ähnliche bekannte Zubereitungsformen.

Die folgenden Ausführungsbeispiele für erfindungsgemäß herstellbare Salze der Verbindung der Formel I, 7-[2-(2-Aminothiazol-4-yl)-2-(Z)-hydroxyimino-acetamido]-3-methoxymethyl-3-cephem-4-carbonsäure-α-(2,2-dimethyl-propanoyloxy)-ethylester, dienen zur weiteren Erläuterung der Erfindung, schränken sie jedoch nicht darauf ein.

## Ausführungsbeispiele

### Ausführungsbeispiel 1

7-[2-(2-Aminothiazol-4-yl)-2-(Z)-hydroxyimino-acetamido]-3-methoxymethyl-3-cephem-4-carbonsäur-α-2,2-dimethyl-propanoyloxy)-ethylester-Benzolsulfonat

Zu einer Lösung von 1,5 g (2,77 mmol) 7-[2-(2-Aminothiazol-4-yl)-2-(Z)-hydroxyimino-acetamido]-3-methoxyethyl-3-cephem-4-carbonsäure-α-(2,2-dimethyl-propanoyloxy)-ethylester in 21 ml Essigester wurde eine Lösung von 0,44 g (1 eq.) Benzolsulfonsäure in 9 ml Essigester getropft. Nach beginnender Kristallisation wurde 30 Minuten nachgerührt, abgesaugt und mit wenig Diethylether nachgewaschen. Nach Trocknen über $CaCl_2$/Paraffin i. Vak. wurden 1,4 g der gewünschten Titelverbindung erhalten, die unter dem Polarisationsmikroskop als kristallines Material identifiziert wurde und lt. HPLC aus einem annähernden 1:1-Gemisch der beiden Diastoreomeren bestand.

Fp.: ab 198°C (unter Zersetzung)

$^1$H-NMR (270 MHz, DMSO-$d_6$): δ(ppm) = 1.15 (9H, 2 x s, tert.-butyl), 1.48 (1H, dd, $CH(CH_3)$), 3.2 (3H, 2 x s, $OCH_3$), 3.47 - 3.7 (2H, 2 x dd, S-$CH_2$), 4.13 (2H, bs, 3'-$CH_2$), 5,21 (1H, 2 x d, J = 6Hz, 6-H), 5.83 (1H, 2 x dd, J = 6Hz, 7-H), 6.8 (1H, 2 x s, Thiazol-H), 6.9 (1H, dq, CH-$CH_3$), 7.3 (3H, m, arom. H), 7.6 (2H, m, arom. H), 9.65 (1H, d, NH), 12.05 (1H, bs, Oxim-H).

Elementaranalyse

| | | | | | | |
|---|---|---|---|---|---|---|
| $C_{27}H_{32}N_5O_{11}S_3$ | ber. | C 46.3 | H 4.8 | N 10.0 | O 25.1 | S 13.8 |
| (699.78) | gef. | C 46.3 | H 4.8 | N 10.1 | O 25.3 | S 13.5 |

### Ausführungsbeispiel 2

7-[2-(2-Aminothiazol-4-yl)-2-(Z)-hydroxyimino-acetamido]-3-methoxymethyl-3-cephem-4-carbonsäure-α-(2,2-dimethyl-propanoyloxy)-ethylester-Toluolsulfonat

Zu einer Lösung von 1,08 g (2 mmol) 7-[2-(2-Aminothiazol-4-yl)-2-(Z)-hydroxyimino-acetamido]-3-methoxymethyl-3-cephem-4-carbonsäure-α-(2,2-dimethyl-propanoyloxy)-ethylester in 3 ml Aceton wurde eine Lösung von 570 mg (3 mmol) p-Toluolsulfonsäure in 1 ml Aceton zugegeben. Anschließend wurden zu der sich

rasch bildenden Kristallsuspension unter Rühren langsam 28 ml Wasser zugetropft. Danach wurde der kristalline Niederschlag abgesaugt, achtmal mit 3 ml Wasser gewaschen und i. Vak. über Calciumchlorid/Paraffin getrocknet. Man erhielt 1,08 g der gewünschten Titelverbindung, die lt. HPLC aus einem annähernden 1 : 1-Gemisch der beiden Diastereomeren bestand und unter dem Polarisationsmikroskop als kristallin charakterisiert wurde.
Fp.: ab 190°C (unter Zersetzung)
$^1$H-NMR (270 MHz, DMSO-$d_6$): δ (ppm) = 1.15 (9H, 2 x s, tert.-butyl), 1.47 (1H, dd, CH-$CH_3$), 2.3 (3H, s, $CH_3$ an Aromat), 3.2 (3H, 2 x s, $OCH_3$), 3.47 - 3.7 (2H, 2 x dd, S-$CH_2$), 4.13 (2H, bs, $CH_2$-$OCH_3$), 5.23 (1H, 2 x d, J = 6Hz, 6-H), 5.83 (1H, 2 x dd, J = 6Hz, 7-H), 6.83 (1H, 2 x s, Thiazol-H), 6.9 (1H, dq, CH-$CH_3$), 7.1 und 7.5 (4H, d, arom. H), 9.68 (1H, d, NH), 12.08 (1H, bs, Oxim-H).

Elementaranalyse

| | | | | | | |
|---|---|---|---|---|---|---|
| $C_{28}H_{36}N_5O_{11}S_3$ | ber. | C 47,1 | H 4,9 | N 9,8 | O 24,7 | S 13,5 |
| (713,80) | gef. | C 47,4 | H 4,9 | N 10,0 | O 24,4 | S 13,1 |

**Ausführungsbeispiel 3**

7-[2-(2-Aminothiazol-4-yl)-2-(Z)-hydroxyimino-acetamido]-3-methoxymethyl-3-cephem-4-carbonsäure-α-(2,2-dimethylpropanoyloxy)ethylester-p-Ethylbenzolsulfonat

Zu einer Lösung von 1,08 g (2 mmol) 7-[2-(2-Aminothiazol-4-yl)-2-(Z)-hydroxyimino-acetamido]-3-methoxymethyl-3-cephem-4-carbonsäure-α-(2,2-dimethylpropanoyloxy)-ethylester in 3 ml Aceton wurde eine Lösung von 664 mg p-Ethylbenzolsulfonsäure (3 mmol) in 1 ml Aceton zugegeben und anschließend unter Rühren langsam 28 ml Wasser zugetropft. Nach erfolgter Zugabe wird angeimpft und 15 Minuten nachgerührt. Den kristallinen Niederschlag saugte man ab, wusch denselben achtmal mit je 3 ml Wasser nach und trocknete i. Vak. über Calciumchlorid/Paraffin. Man erhielt 1,28 g der gewünschten Titelverbindung, die lt. HPLC aus einem annähernden 1 : 1-Gemisch der beiden Diastereomeren bestand und unter dem Polarisationsmikroskop als kristallin charakterisiert wurde.
Fp.: ab 170°C (unter Zersetzung)
$^1$H-NMR (270 MHz, DMSO-$d_6$): δ (ppm) = 1.15 (9H, 2 x s, tert.-butyl), 1.17 (3H, t, $CH_3CH_2$-), 1.5 (3H, dd, CH-$CH_3$), 2.6 (2H, q, $CH_3CH_2$-), 3.2 (3H, 2 x s, $OCH_3$), 3.47 - 3.7 (2H, 2 x dd, S-$CH_2$), 4.13 (2H, d, $CH_2OCH_3$), 5.23 (1H, 2 x d, J = 6Hz, 6-H), 5.83 (1H, 2 x dd, J = 6Hz, 6-H), 5.83 (1H, 2 x dd, J = 6Hz, 7-H), 6.83 (1H, 2 x s, Thiazol-H), 6.9 (1H, dq, CH-$CH_3$), 7.15 und 7.51 (4H, d, arom. H), 9.67 (1H, d, NH), 12.05 (1H, bs, Oxim-H).

Elementaranalyse

| | | | | | | |
|---|---|---|---|---|---|---|
| $C_{29}H_{37}N_5O_{11}S_3$ | bei: | C 47,9 | H 5,1 | N 9,6 | O 24,2 | S 13,2 |
| (727,86) | gef.: | C 47,6 | H 5,2 | N 9,8 | O 24,0 | S 13,3 |

Ausführungsbeispiel 4

7-[2-(2-Aminothiazol-4-yl)-2-(Z)-hydroxyimino-acetamido]-3-methoxymethyl-3-cephem-4-carbonsäure-α-(2,2-dimethylpropanoyloxy)ethylester-Toluolsulfonat

Zu einer Lösung von 5 g (9,3 mmol) 7-[2-(2-Aminothiazol-4-yl)-2-(Z)-hydroxyimino-acetamido]-3-methoxymethyl-3-cephem-4-carbonsäure-α-(2,2-dimethyl- propanolyoxy)ethylester in 32 ml Ethanol wurde eine Lösung von 2,1 g (11 mmol) p-Toluolsulfonsäure in 5 ml Ethanol getropft. Nach 30-minütigem Rühren bei Raumtemperatur wurden zu dem gebildeten Kristallbrei insgesamt 280 ml Diisopropylether zugegeben und kurz nachgerührt. Danach saugte man den gebildeten Niederschlag ab, wusch mit wenig Diisopropylether nach und trocknete den Rückstand i. Vak. über Calciumchlorid/Paraffin. Man erhielt 5,4 g der gewünschten kristallinen Titelverbindung, die lt. HPLC aus einemannähernden 1: 1-Gemisch der beiden Diastereomeren bestand und in ihren physikalischen und chemischen Eigenschaften mit dem Produkt aus dem Ausführungsbeispiel 2 identisch war.

Fp.: ab 190°C (unter Zersetzung)

Ausführungsbeispiel 5

7-[2-(2-Aminothiazol-4-yl)-2-(Z)-hydroxyimino-acetamido]-3-methoxymethyl-3-cephem-carbonsäure-α-( 2,2-dimethylpropanoyloxy)ethylester-Toluolsulfonat

Zu einer Lösung von 5 g (9,3 mmol) 7-[2-(2-Aminothiazol-4-yl)-2-(Z)-hydroxyimino-acetamido]-3-methoxymethyl-3-cephem-4-carbonsäure-α-(2,2-dimethyl-dimethyl-propanoyloxy)ethylester in 35 ml Aceton wurde eine Lösung von 1,8 g (15 mmol) p-Toluolsulfonsäure in 5 ml Aceton getropft. Nach kurzem Rühren bildete sich ein dicker Niederschlag. Nach 15 Minuten Rühren wurden zur Vervollständigung der Kristallisation insgesamt 185 ml Diisopropylether langsam zugetropft und kurze Zeit nachgerührt. Danach saugte man den Niederschlag ab, wusch mit wenig Diisopropylether nach und trocknete i. Vak. über Calciumchlorid/Paraffin. Man erhielt 5,9 g kristalline Titelverbindung, die lt. HPLC aus einem annähernden 1:1-Gemisch der beiden Diastereomeren bestand und in ihren physikalischen und chemischen Eigenschaften mit dem Produkt aus Ausführungsbeispiel 2 identisch war.
Fp.: ab 190°C (unter Zersetzung)

Ausführungsbeispiel 6

7-[2-(2-Aminothiazol-4-yl)-2-(Z)-hydroxyimino-acetamido]-3-methoxymethyl-3-cephem-4-carbonsäure-α-(2,2-dimethylpropanoyloxy)ethylester-Toluolsulfonat

Zu einer Lösung von 5 g (9,3 mmol) 7-[2-(2-Aminothiazol-4-yl)-2-(Z)-hydroxyimino-acetamido]-3-methoxymethyl-3-cephem-4-carbonsäure-α-(2,2-dimethyl-propanoyloxy)-ethylester in 35 ml Aceton wurde eine Lösung von 2,1 g (11 mmol) p-Toluolsulfonsäure in 5 ml Aceton zugetropft. Nach kurzem Rühren bildete sich ein Niederschlag, der durch langsame Zugabe von 250 ml t-Butylmethyl-ether vervollständigt wurde. Nach 15 Minuten Nachrühren wurde das kristalline Produkt abgesaugt, mit wenig t-Butylmethylether nachgewaschen und i. Vak. über Calciumchlorid/Paraffin getrocknet. Man erhielt 4,6 g der gewünschten kristallinen Titelverbindung, die lt. HPLC aus einem annähernden 1:1-Verhältnis der beiden Diestereomeren bestand und in ihren physikalischen und chemischen Eigenschaften mit dem Produkt aus Ausführungsbeispiel 2 identisch war.
Fp.: ab 190°C (unter Zersetzung)

## Patentansprüche

### Patentansprüche für folgende Vertragsstaaten : AT, BE, CH, DE, DK, FR, GB, IT, LI, LU, NL, SE

1. Kristalline Cephem-Säureadditionssalze der allgemeinen Formel II

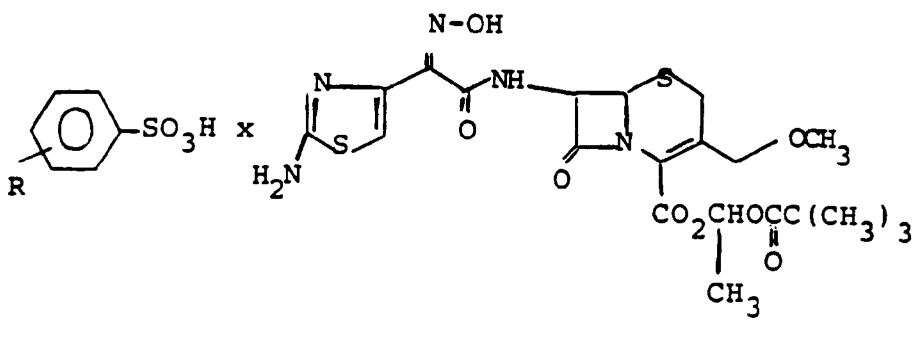

(II)

in der R für Wasserstoff oder $C_1$-$C_4$-Alkyl und die Gruppe =N-OH in syn-Position steht.

2. 7-[2-(2-Aminothiazol-4-yl)-2-(Z)-hydroxyimino-acetamido]-3-methoxymethyl-3-cephem-4-carbonsäure-α-(2,2-dimethyl-propanoyloxy)-ethylester-Benzolsulfonat.

3. 7-[2-(2Aminothiazol-4-yl)-2-(Z)-hydroxyimino-acetamido]-3-methoxymethyl-3-cephem-4-carbonsäure-α-(2,2-dimethyl-propanoyloxy)-ethylester-Toluolsulfonat.

4. 7-[2-(2-Aminothiazol-4-yl)-2-(Z)-hydroxyimino acetamido]-3-methoxymethyl-3-cephem-4-carbonsäure-α-(2,2-dimethylpropanoyloxy)-ethylester-p-Ethylbenzolsulfonat.

5. Verfahren zur Herstellung von kristallinen Cephem-Säureadditionssalzen der allgemeinen Formel II

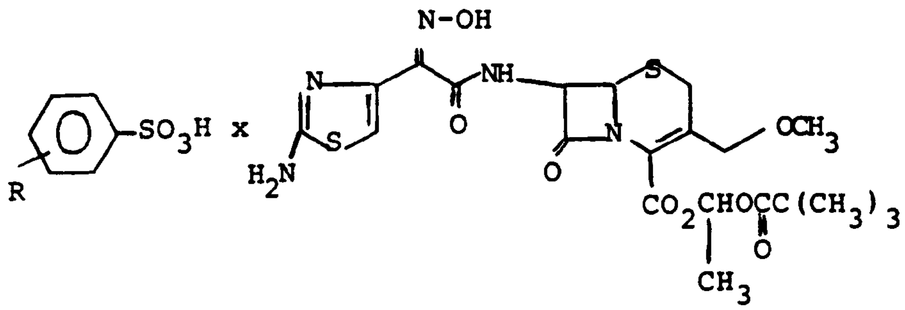

(II)

in der R für Wasserstoff oder $C_1$-$C_4$-Alkyl und die Gruppe =N-OH in syn-Position steht, dadurch gekennzeichnet, daß man ein amorphes Cephem der allgemeinen Formel I

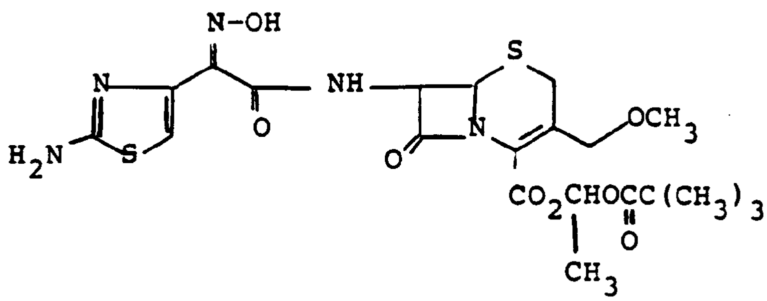

(I)

mit einer Säure der allgemeinen Formel III

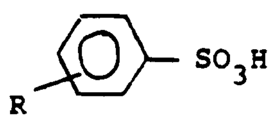

(III)

in der R für Wasserstoff oder $C_1$-$C_4$-Alkyl steht, in einem organischem Lösungsmittel umsetzt und das Säureadditionssalz direkt oder durch Zugabe eines anderen organischen Lösungsmittels oder von Wasser kristallisieren läßt.

6. Gegen bakterielle Infektionen wirksame pharmazeutische Zubereitungen gekennzeichent durch einen Gehalt an Cephem-Säureadditionssalzen der allgemeinen Formel II.

7. Verfahren zur Herstellung von gegen bakterielle Infektionen wirksamen pharmazeutischen Zubereitungen, dadurch gekennzeichnet, daß ein Cephem-Säureadditionssalz der allgemeinen Formel II mit pharmazeutisch üblichen Trägerstoffen oder Verdünnungsmitteln in eine pharmazeutisch geeignete Verabreichungsform gebracht wird.

8. Verwendung von Cephem-Säureadditionssalzen der allgemeinen Formel II zur Herstellung eines Arzneimittels zur Bekämpfung bakterieller Infektionen.

**Patentansprüche für folgende Vertragsstaaten : ES, GR**

1. Verfahren zur Herstellung von kristallinen Cephem-Säureadditionssalzen der allgemeinen Formel II

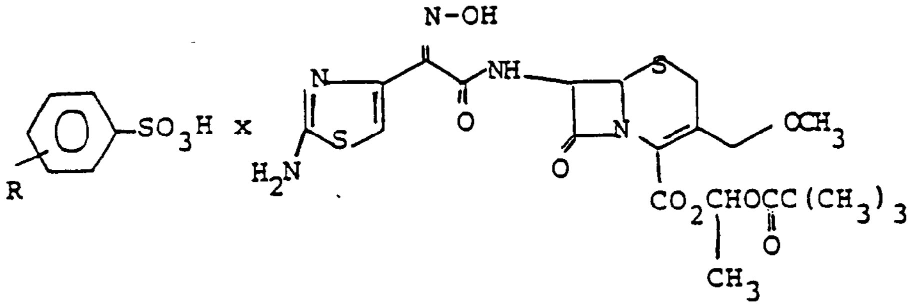

(II)

in der R für Wasserstoff oder $C_1$-$C_4$-Alkyl und die Gruppe =N-OH in syn-Position steht, dadurch gekennzeichnet, daß man ein amorphes Cephem der allgemeinen Formel I

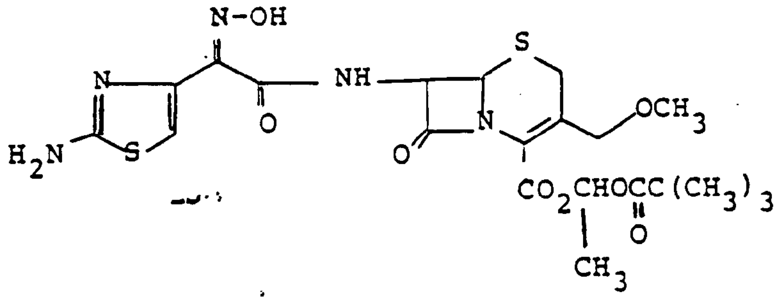

(I)

mit einer Säure der allgemeinen Formel III

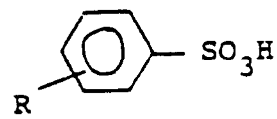

(III)

in der R für Wasserstoff oder $C_1$-$C_4$-Alkyl steht, in einem organischem Lösungsmittel umsetzt und das Säureadditionssalz direkt oder durch Zugabe eines anderen organischen Lösungsmittels oder von Wasser kristallisieren läßt.

## Claims

### Claims for the following Contracting States : AT, BE, CH, DE, DK, FR, GB, IT, LI, LU, NL, SE

1. A crystalline cephem acid addition salt of the formula II

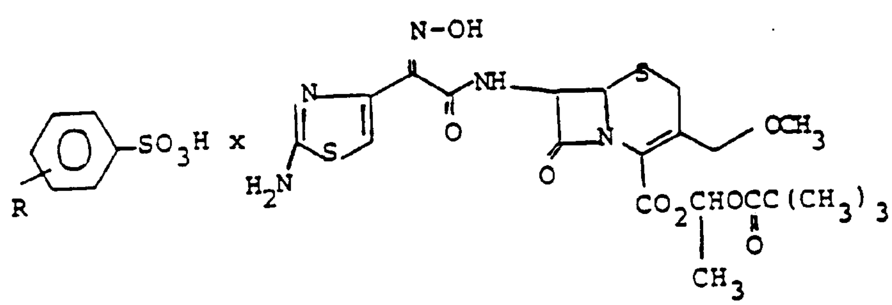

(II)

in which R stands for hydrogen or $C_1$-$C_4$-alkyl and the group =N-OH is in the syn-position.

2. α-(2,2-Dimethylpropanoyloxy)ethyl 7-[2-(2-aminothia-zol-4-yl)-2-(Z)-hydroxyimino-acetamido]-3-methoxymethyl-3-cephem-4-carboxylate benzenesulfonate.

3. α-(2,2-Dimethylpropanoyloxy)ethyl 7-[2-(2-aminothia-zol-4-yl)-2-(Z)-hydroxyimino-acetamido]-3-methoxymethyl-3-cephem-4-carboxylate toluenesulfonate.

4. α-(2 ,2-Dimethylpropanoyloxy) ethyl 7-[2-(2-aminothia-zol-4-yl)-2-(Z)-hydroxyimino-acetamido]-3-methoxymethyl-3-cephem-4-carboxylate p-ethylbenzene-sulfonate.

5. A process for the preparation of crystalline cephem acid addition salts of the formula II

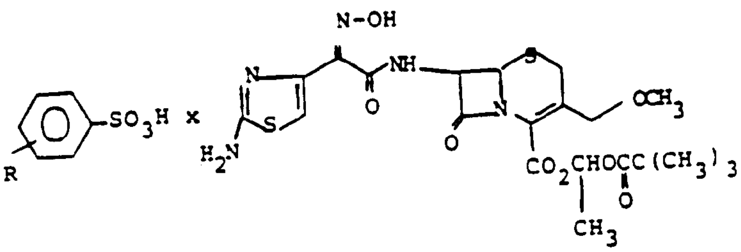

(II)

in which R stands for hydrogen or $C_1$-$C_4$-alkyl and the group =N-OH is in the syn-position, which comprises reacting an amorphous cephem of the formula I

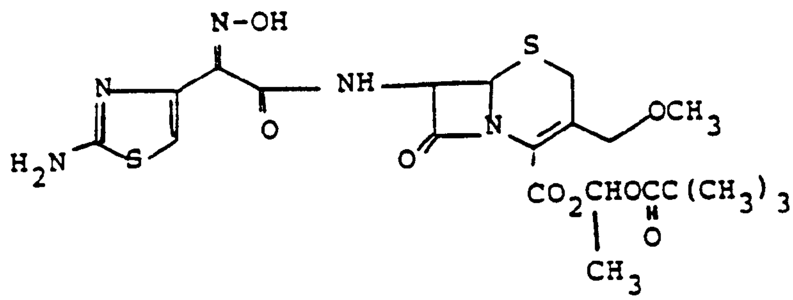

(I)

with an acid of the formula III

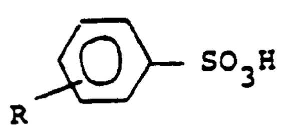

(III)

in which R stands for hydrogen or $C_1$-$C_4$-alkyl, in an organic solvent and allowing the acid addition salt to crystallize directly or by addition of another organic solvent or water.

6. A pharmaceutical preparation effective against bacterial infections which contains a cephem acid addition salt of the formula II.

7. A process for the preparation of pharmaceutical preparations effective against bacterial infections, wherein a cephem acid addition salt of the formula II is brought into a suitable form for pharmaceutical administration using customary pharmaceutical excipients or diluents.

8. The use of cephem acid addition salts of the formula II for preparing a pharmaceutical combating bacterial infections.

**Claims for the following Contracting States : ES, GR**

1. A process for the preparation of crystalline cephem acid addition salts of the formula II

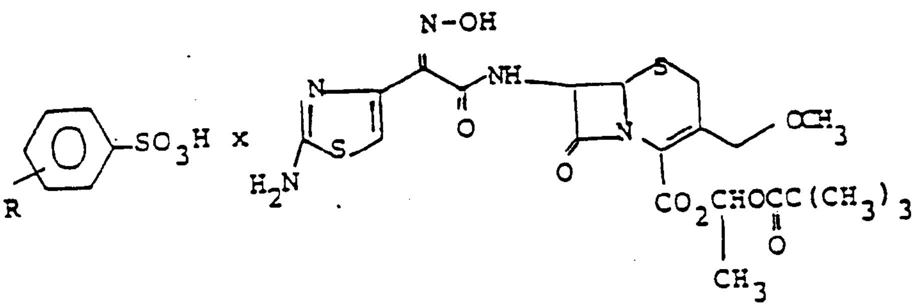

(II)

in which R stands for hydrogen or $C_1$-$C_4$-alkyl and the group =N-OH is in the syn-position, which comprises reacting an amorphous cephem of the formula I

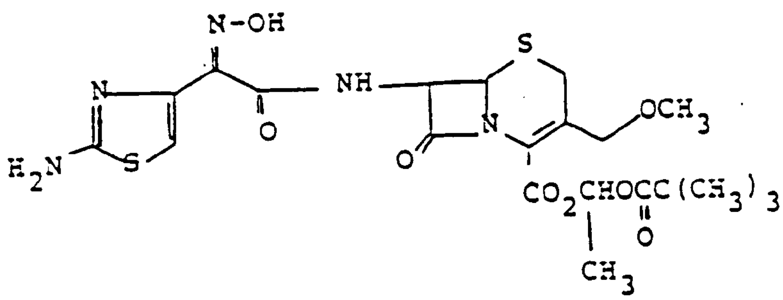

(I)

with an acid of the formula III

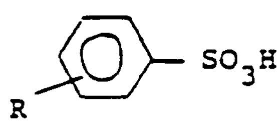

(III)

in which R stands for hydrogen or $C_1$-$C_4$-alkyl, in an organic solvent and allowing the acid addition salt to crystallize directly or by addition of another organic solvent or water.

**Revendications**

**Revendications pour les Etats contractants suivants : AT, BE, CH, DE, DK, FR, GB, IT, LI, LU, NL, SE**

1. Sels d'addition cristallins d'acide et de céphème de formule générale II:

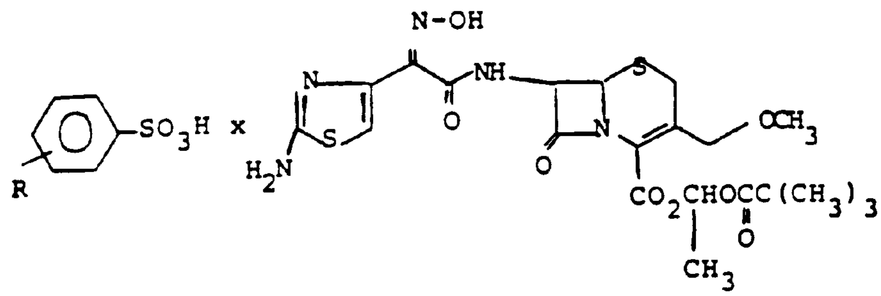

(II)

dans laquelle R représente l'atome d'hydrogène ou un groupe alkyle en $C_{1-4}$ et le groupe =N-OH est en position syn.

2. Benzènesulfonate de 7-[2-(2-aminothiaksl-4-y1)-3-(Z)-hydroxyimino-acétamido]-3-methoxyméthyl-3-céphème-4-carboxylate de α-(2,2-diméthylpropanoyloxy)éthyle.

3. Toluènesulfonate de 7-[2-(2-aminothiazol-4-yl)-2-(Z)-hydroxyimino-acétamido]-3-methoxymethyl-3-céphème-4-carboxylate de α-(2, 2-diméthylpropanoyloxy)éthyle.

4. p-Ethylbenzènesulfonate de 7-[2-(2-aminothiazol-4-yl)-2-(Z)-hydroxyimino-acétamido]-3-méthOxyméthyl-3-céphème-4-carboxylate de α-(2,2-diméthylprepanoyloxy)éthyle.

5. Procédé de préparation de sels d'addition cristallins d'acide et de céphème de formule générale II:

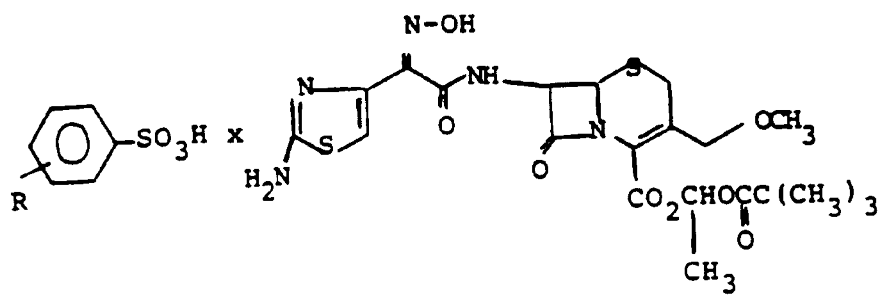

(II)

dans laquelle R représente l'atome d'hydrogène ou un groupe alkyle en $C_{1-4}$ et le groupe =N-OH est en position syn, caractérisé en ce qu'on fait réagir un céphéme amorphe de formule générale I:

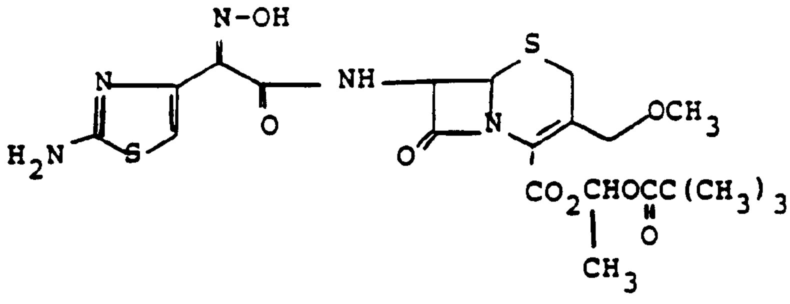

(I)

avec un acide de formule générale III:

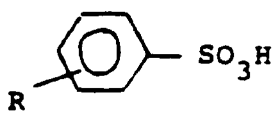

dans laquelle R représente l'atome d'hydrogène ou un groupe alkyle en $C_{1-4}$, dans un solvant organique, et on fait cristalliser le sel d'addition d'acide soit directement, soit en ajoutant un autre solvant organique, soit encore dans l'eau.

6. Préparations pharmaceutiques actives contre les infections bactériennes, caractérisées en ce qu'elles contiennent des sels d'addition d'acide et de céphème de formule générale II.

7. Procédé de fabrication de préparations pharmaceutiques actives contre les infections bactériennes, caractérisé en ce qu'on met un sel d'addition d'acide et de céphème, de formule générale II, et les véhicules ou diluants pharmaceutiques usuels, sous une forme d'administration pharmaceutiquement acceptable.

8. Utilisation de sels d'addition d'acide et de céphème de formule générale II pour la fabrication d'un médicament pour lutter contre les infections bactériennes.

**Revendications pour les Etats contractants suivants : ES, GR**

1. Procédé de préparation de sels d'addition cristallins d'acide et de céphème de formule générale II:

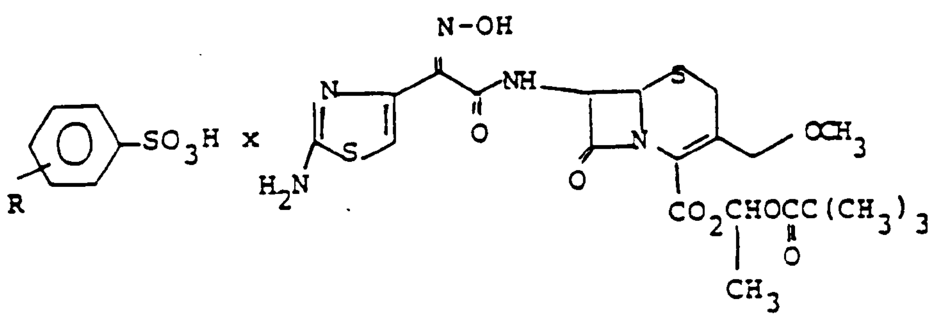

(II)

dans laquelle R représente l'atome d'hydrogène ou un groupe alkyle en $C_{1-4}$ et le groupe =N-OH est en position syn, caractérisé en ce qu'on fait réagir un céphème amorphe de formule générale I:

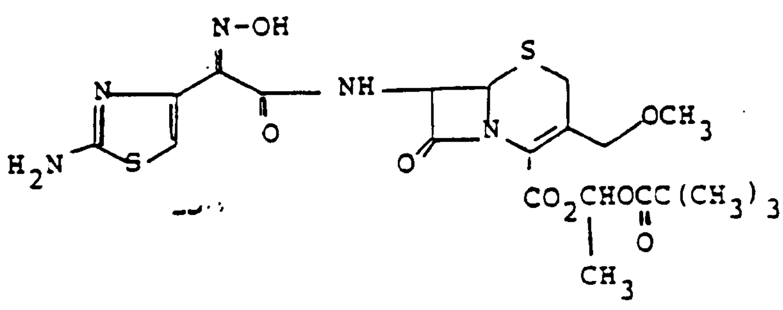

(I)

avec un acide de formule générale III:

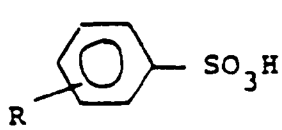

dans laquelle R représente l'atome d'hydrogène ou un groupe alkyle en $C_{1-4}$, dans un solvant organique,

et on fait cristalliser le sel d'addition d'acide soit directement, soit en ajoutant un autre solvant organique, soit encore dans l'eau.